# FASCICULE DE BREVET EUROPEEN

(11) **EP 3 413 929 B1**
(45) Date de publication et mention de la délivrance du brevet: **02.08.2023**
(21) Numéro de dépôt: 17707387.1
(22) Date de dépôt: 08.02.2017
(51) Int. Cl.: A61L 2/18, B08B 3/10

(54) **DISPOSITIF DE NETTOYAGE D'UNE PROTHÈSE AUDITIVE**
VORRICHTUNG ZUR REINIGUNG EINES HÖRGERÄTS
DEVICE FOR CLEANING A HEARING AID

(30) Priorité: 08.02.2016 FR 1650974; 08.02.2016 FR 1650977
(43) Date de publication de la demande: 19.12.2018
(73) Titulaire: MG Developpement, 34470 Perols (FR)
(72) Inventeur: GIL, José, 34470 Perols (FR)
(74) Mandataire: Rhein, Alain
(86) Numéro de dépôt international: PCT/FR2017/050280
(87) Numéro de publication internationale: WO 2017/137695

(56) Documents cités:
- US-A1- 2008 253 579
- US-A1- 2010 189 598
- US-A1- 2013 220 385

## Description

La présente invention entre dans la domaine du nettoyage d'une prothèse auditive.

Porté quotidiennement, un embout intra-auriculaire d'une prothèse auditive est régulièrement souillé par du cérumen et/ou des impuretés. Or, un embout intra-auriculaire d'une prothèse auditive souillé présente une perte d'efficacité et est susceptible de créer des irritations dans le conduit auditif. Afin de garantir le bon fonctionnement de la prothèse auditive et le confort de la personne qui la porte, il est nécessaire de procéder à un nettoyage régulier de cet embout.

On connaît au moins deux types d'embout intra-auriculaire de prothèse auditive, un premier type d"embout intra-auriculaire comporte des éléments électroniques permettant de réaliser la transmission ou l'amplification des sons perçue par la prothèse. à cet effet, l'embout intra-auriculaire comprend une partie distale destinée à être au contact avec le conduit auditif et une partie proximale raccordant l'embout intra-auriculaire à un contour d'oreille. Le contour d'oreille, destiné à être placé derrière l'oreille d'un utilisateur, comporte des moyens d'alimentation électriques, un interrupteur etc.

Un second type d'embout intra-auriculaire comporte tous les éléments techniques et électroniques d'une prothèse auditive, l'embout intra-auriculaire constitue donc à lui seul la prothèse auditive et n'a pas besoin d'être relié à un contour d'oreille.

De manière générale, les éléments électroniques étant susceptibles d'être endommagés lors d'une exposition prolongée à un liquide de nettoyage (par exemple de l'eau, une solution céruménolytique comportant des propriétés désinfectantes etc.), il est préférable d'éviter de réaliser le nettoyage de l'embout par immersion complète dans un liquide de nettoyage. C'est d'autant plus le cas pour le second type d'embout intra-auriculaire qui comporte un plus grand nombre d'éléments électroniques que le premier.

Dans cette optique, il existe un dispositif de nettoyage décrit dans le document FR 2 966 046, mettant en œuvre un procédé de nettoyage par effleurement de l'embout en générant un vortex de liquide autour de l'embout. à cet effet, la dispositif de nettoyage comprend un logement adapté à maintenir l'embout dans une cavité de nettoyage. La cavité de nettoyage est réalisée de manière à générer un vortex de liquide autour de l'embout lorsqu'il se trouve dans son logement. A ces fins, la cavité de nettoyage comprend une buse d'injection de liquide adaptée à injecter du liquide dans la cavité de nettoyage, des moyens d'orientation de liquide (par exemple des rainures ou des ailettes) permettant d'orienter le flux de liquide, et un orifice d'évacuation du liquide situé en vis-à-vis du logement et ayant pour effet de centrer l'œil du vortex sous l'embout de la prothèse.

Toutefois, un tel dispositif de nettoyage présente l'inconvénient de posséder une structure complexe, rendant son industrialisation difficile.

De plus, le logement accueillant l'embout intra-auriculaire doit être parfaitement ajusté à un tel embout pour que n'émerge, au travers de l'ouverture sous le logement, que la partie distale à nettoyer et éviter toute remontée de liquide de nettoyage, au travers de cette ouverture au niveau de la partie proximale.

Or, il convient de rappeler qu'une grande variété d'embouts intra-auriculaires est disponible sur le marché, des embouts qui se distinguent par leur diversité de forme déterminée par celle du conduit auditif de l'utilisateur, afin de garantir au mieux son confort lors du port de la prothèse auditive. Il est donc nécessaire, pour le fabricant de tels dispositifs de nettoyage de proposer pour ses appareils une multiplicité de logements différents, de forme strictement adaptée à cette diversité de formes d'embouts. Ceci génère inévitablement des frais de fabrication supplémentaires se répercutant sur le coût final de l'appareil de nettoyage. De plus, dans certains cas extrêmes, il peut s'avérer qu'aucun des différents logements proposés par le fabricant de l'appareil de nettoyage ne convienne. Il peut également arriver que l'utilisateur remplace un embout auditif par un autre embout de forme différente, auquel cas la forme du logement prévu initialement ne correspond donc plus. L'utilisateur peut ainsi se voir parfois contraint d'aller faire mouler un logement sur mesures à la forme précise de l'embout par ses propres moyens, et de prendre à sa charge l'ensemble des frais liés à une telle réalisation. Ceci est non seulement contraignant mais également particulièrement onéreux.

La présente invention se veut à même de répondre à ces inconvénients au travers d'une solution technique simple, d'un coût de fabrication réduit et, qui plus est, évitant la nécessité d'un ajustement strict du logement de réception de l'embout à la forme spécifique de ce dernier.

On connaît également le document US2008253579 A1, qui décrit un appareil de nettoyage alternatif d'une prothèse auditive.

Dans cette optique, la présente invention comporte une cavité de nettoyage universelle dans laquelle tous types d'embouts intra-auriculaires peuvent être insérés. Toutefois, le nettoyage d'un embout intra-auriculaire de forme spécifique dans une cavité de nettoyage universelle par effleurement d'un vortex de liquide pose un nouveau problème technique : comment créer un vortex de liquide au sein d'une cavité de nettoyage universelle permettant de réaliser un nettoyage par effleurement d'embouts intra-auriculaires de toutes formes, tout en protégeant efficacement chaque type d'embout et notamment leur partie proximale des projections de liquide inhérentes à la formation d'un vortex de liquide et à l'instabilité d'un vortex de liquide.

A cet effet, la présente invention entend proposer une solution permettant de gérer le flux de liquide de nettoyage qui est injecté dans la cavité de nettoyage, afin d'assurer le nettoyage de la partie distale de l'embout par effleurement d'un vortex liquide, sans risque d'éclaboussures substantielles de la partie proximale de l'embout et de submersion de l'embout.

Ainsi, c'est au travers d'une démarche inventive qu'il a en particulier été imaginé que l'injection de liquide de nettoyage intervienne au niveau d'une peau externe ceinturant une peau interne qui délimite la cavité de nettoyage et protège parfaitement l'embout de toutes projections de liquide de nettoyage, la peau interne délimitant à sa base une ouverture permettant le passage du liquide depuis la peau externe vers la cavité de nettoyage. Ainsi, un déplacement de liquide est généré au sain de la cavité de nettoyage et permet de nettoyer l'embout intra-auriculaire par effleurement de liquide de nettoyage. De préférence, le déplacement de liquide de nettoyage est circulaire et forme un vortex autour de l'extrémité distale de l'embout.

à ce titre, un premier aspect de l'invention concerne un dispositif de nettoyage d'une prothèse auditive par effleurement d'un liquide, tel que défini dans la revendication 1.

Avantageusement, l'injection de liquide de nettoyage intervient au niveau de la peau externe, alors que la peau interne protège parfaitement l'embout de toutes projections de liquide de nettoyage, et permet de générer un flux de liquide en déplacement vers l'embout, ce qui favorise un nettoyage de l'embout par effleurement et facilitant à la dissolution de cérumen présent à la surface de l'embout.

Selon une première caractéristique du premier aspect de l'invention, la peau externe comporte à un niveau supérieur aux moyens d'injection un orifice d'évacuation latéral permettant d'évacuer un excès de liquide circulant entre les peaux interne et externe. Cet orifice latéral joue un rôle de trop-plein et permet de contrôler la hauteur maximale de liquide de nettoyage entre les peaux interne et externe, et par voie de faits dans la cavité de nettoyage. De manière avantageuse, l'orifice latéral contrôle la hauteur du flux de liquide, ce qui permet d'éviter tout risque de submersion de l'embout.

Selon une deuxième caractéristique du premier aspect de l'invention, les moyens d'injection de liquide sont tangents à la peau externe de façon à projeter tangentiellement le liquide le long de la peau externe. Cette caractéristique contribue à la création du flux de liquide entre les peaux interne et externe, protégeant ainsi l'embout de toutes projections de liquide.

Selon une troisième caractéristique du premier aspect de l'invention, le dispositif de nettoyage comprend un réservoir équipé d'un bouchon mobile entre une position fermée dans laquelle le liquide est cantonné dans le réservoir, une position ouverte dans laquelle le liquide circule entre la cavité de nettoyage et le réservoir, et une position de vidange dans laquelle la liquide est vidangé depuis le réservoir hors du dispositif de nettoyage. Cette caractéristique permet à l'utilisateur du dispositif de nettoyage, de le transporter réservoir plein lorsque le bouchon est en position formée.

Selon une quatrième caractéristique du premier aspect de l'invention, le fond comprend des moyens de réception de l'embout saillants et adaptés à recevoir l'embout de façon surélevée par rapport au fond. Cette caractéristique a pour effet de surélever l'embout intra-auriculaire par rapport au fond de la cavité de nettoyage et contribue ainsi, d'une part, à positionner correctement l'embout au sein de la cavité, et d'autre part, à éviter que le flux de liquide ne submerge l'embout.

Selon une particularité de la quatrième caractéristique du premier aspect de l' invention, les moyens de réception sont formés par une barre radiale.

Les peaux interne et externe forment entre elles un espace annulaire qui est fermé supérieurement.

Selon une caractéristique du premier aspect de l'invention, la peau externe comprend des moyens de projection d'air dans l'espace annulaire. Cette caractéristique permet de sécher rapidement l'embout intra-auriculaire après un cycle de nettoyage. De plus, le fait que les moyens de projection d'air se trouvent entre la peau externe et la peau interne, permet de mettre l'air en circulation tourbillonnaire et ainsi d'optimiser le séchage de l'embout intra-auriculaire suite à un cycle de nettoyage.

Selon une particularité du premier aspect de l'invention, le dispositif de nettoyage comprend des moyens de chauffage chauffant la flux l'air projeté.

Selon une caractéristique du premier aspect de l'invention, le dispositif de nettoyage comprend une lampe UVC adaptée à diffuser des ondes UVC en direction de la prothèse auditive de manière à réaliser une désinfection par irradiation UVC.

Selon une caractéristique du premier aspect de l'invention, le réservoir comporte des moyens de détection du niveau du liquide, ces moyens de détection étant adaptés à déclencher l'émission d'un signal d'avertissement lorsque le niveau de liquide, présent à l'intérieur du réservoir, atteint un niveau seuil prédéfini.

Un second aspect de l'invention concerne un ensemble comprenant un dispositif de nettoyage selon le premier aspect de l'invention et une unité de rechargement de liquide de nettoyage adaptée à remplir un réservoir du dispositif de nettoyage.

Avantageusement, l'unité de rechargement comporte un manchon adapté coopérer avec au moins une cavité de nettoyage du dispositif de nettoyage de manière à transférer le liquide de nettoyage depuis l'unité de rechargement vers le réservoir du dispositif de nettoyage.

La coopération entre un manchon de l'unité de rechargement et la cavité de nettoyage du dispositif de nettoyage favorise un transfert du liquide de nettoyage par gravitation depuis l'unité de rechargement vers le réservoir du dispositif de nettoyage par gravitation. De plus, cette coopération permet un remplissage du réservoir propre, sans éclaboussure particulièrement adapté à des personnes présentant une dextérité réduite comme des personnes âgées ou handicapées.

Selon un premier mode de réalisation du second aspect de l'invention, l'unité de rechargement comporte, d'une part, une cuve équipée d'une ouverture fermée par un opercule, et d'autre part, des moyens de connexion qui sont adaptés à perforer l'opercule lorsqu'ils sont mis en connexion avec une cavité de nettoyage du dispositif de nettoyage.

Selon un deuxième mode de réalisation du second aspect de l'invention, l'unité de rechargement comporte un capuchon qui comprend un canal, un perforateur adapté à perforer l'opercule et des moyens de connexion avec la cavité de nettoyage, le capuchon étant mobile par rapport à la cuve entre une position préliminaire dans laquelle le perforateur est écarté de l'ouverture, et une position de percussion dans laquelle le perforateur perfore l'opercule et libère le liquide par le canal.

Avantageusement, cette unité de rechargement permet lorsque la capuchon est maintenu en position préliminaire de conserver hermétiquement le liquide à l'intérieur de la cuve, et de maîtriser tant la perforation de l'opercule que l'écoulement du liquide qui s'en suit. De plus, son utilisation permet de limiter le volume du dispositif de nettoyage, et donc l'encombrement de celui-ci puisqu'il n'est plus nécessaire de prévoir au sein de celui-ci un espace suffisamment grand pour accueillir l'unité de rechargement.

Selon un troisième mode de réalisation du second aspect de l'invention, le perforateur définit une canule d'écoulement du liquide de nettoyage contenu dans la cuve au travers du canal du capuchon jusqu'à la cavité de nettoyage. Cette caractéristique permet de limiter la débit de liquide lors de son écoulement hors de l'unité de rechargement, évitant ainsi toutes éclaboussures intempestives.

Selon un quatrième mode de réalisation du second aspect de l'invention, le perforateur s'étend dans le sens de déplacement du capuchon et présente deux faces latérales reliées entre elles par une base comportant une pointe perforatrice apte à perforer l'opercule.

Selon un cinquième mode de réalisation du second aspect de l'invention, le perforateur compartimente le canal du capuchon en une zone centrale située entre deux faces latérales du perforateur, et deux zones latérales situées chacune entre une face latérale et un bord du canal. Cette caractéristique favorise un remplissage propre du réservoir du dispositif de nettoyage, les zones latérales réalisant un appel d'air qui permet au liquide de s'écouler par gravitation au travers de la zone centrale correspondant à la canule d'écoulement défini par le perforateur.

D'autres particularités et avantages de la présente invention apparaîtront au cours de la description détaillée qui suit d'un exemple de réalisation donné à titre indicatif et non limitatif.

La compréhension de cette description sera facilitée en se référant aux sept dessins placés en annexe dans lesquels :
- la figure 1 est une représentation schématisée en coupe d'une cavité de nettoyage du dispositif de nettoyage selon l'invention, la cavité de nettoyage contenant un embout intra-auriculaire ;
- la figure 2 est une représentation tridimensionnelle de la cavité de nettoyage de la figure 1 ;
- la figure 3 est vue de haut d'un espace de nettoyage d'un dispositif de nettoyage conforme à l'invention ;
- la figure 4 est une vue d'une coupe transversale du dispositif de nettoyage conforme à l'invention, dans laquelle les flèches illustrent la circulation du flux de liquide lors d'un nettoyage de l'embout;
- la figure 5 est une vue en coupe longitudinale du dispositif de nettoyage de la figure 4, dans laquelle les flèches illustrent la circulation de flux de liquide lors d'un nettoyage de l'embout ;
- la figure 6 est une vue en coupe transversale du dispositif de nettoyage de la figure 4, dans laquelle les flèches illustrent la circulation du flux d'air lors du séchage de l'embout ;
- la figure 7 est une vue d'une coupe longitudinale du dispositif de la figure 4, dans laquelle les flèches illustrent la circulation du flux d'air lors d'un séchage de l'embout ;
- la figure 8 est une représentation en perspective, avant remplissage, d'un dispositif de nettoyage illustré aux figures 4 à 7 et d'une unité de rechargement conforme à l'invention, dans laquelle le capuchon est placé en position préliminaire ;
- la figure 9 est une vue de dessous de l'unité de rechargement de la figure 8 ;
- la figure 10 est une vue de face de l'unité de chargement de la figure 8, le capuchon étant en position de percussion ;
- la figure 11 est une vue de face en perspective du capuchon de l'unité de rechargement de la figure 8 ;
- la figure 12 est une vue de dessus du capuchon de l'unité de rechargement de la figure 8 ; et
- la figure 13 est une vue de dessous de la cuve d'une unité de rechargement de la figure 8.

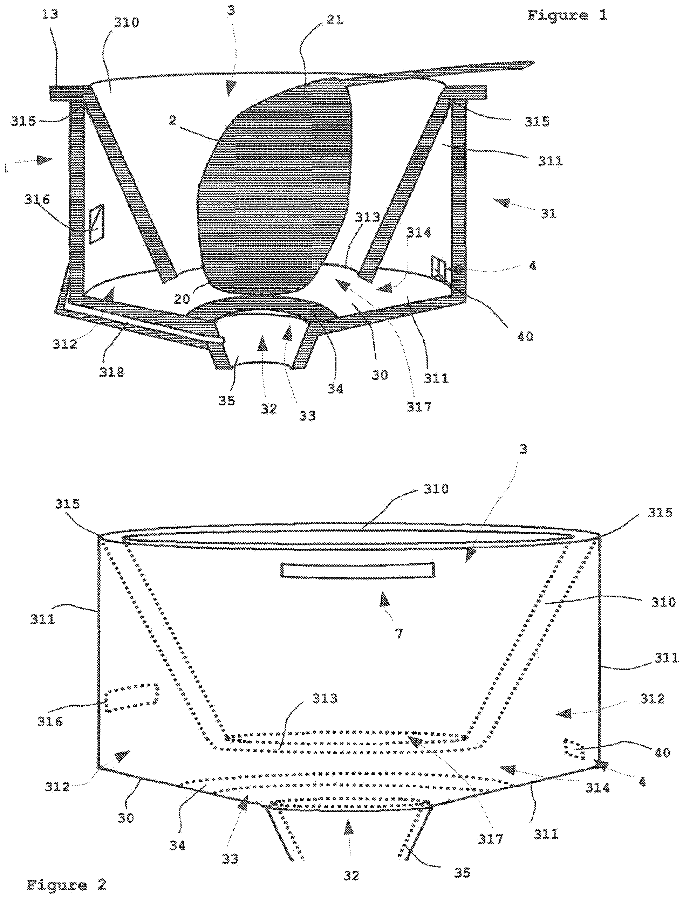

L'invention concerne un dispositif de nettoyage 1, destiné au nettoyage d'au moins prothèse auditive 20 et notamment de son embout intra-auriculaire 2 (illustré à la figure 1).

La prothèse auditive 20 et plus particulièrement l'embout intra-auriculaire 2 comporte un certain nombre de composants électroniques d'amplification et/ou de transmission. L'embout intra-auriculaire 2 présente une partie distale 21 prévue pour venir s'engager dans le conduit auditif d'un utilisateur et une partie proximale 22 venant généralement se positionner à l'avant du conduit auditif. Dans le présent exemple, la partie proximale 22 relie, l'embout intra-auriculaire 2 à un contour d'oreille 23 porté par le contour de l'oreille d'un utilisateur. Toutefois, le dispositif selon l'invention est également prévu pour nettoyer un embout intra-auriculaire du second type (sans contour d'oreille).

Le dispositif de nettoyage 1 est adapté à effectuer un nettoyage de la prothèse auditive 20, et plus particulièrement de l'embout intra-auriculaire 2, et encore plus particulièrement, de la partie distale 21 par effleurement d'un vortex de liquide. Le liquide effleurant la partie distale 21, détache le cérumen et d'autres impuretés de l'embout intra-auriculaire 2 par dissolution. Il est à noter que certains liquides de nettoyage (céruménolytique) ont également des propriétés désinfectantes afin d'éliminer des irritants ou allergisants se trouvant à la surface de l'embout intra-auriculaire 2.

à cet effet, le dispositif de nettoyage 1 comporte un espace de nettoyage 10 situé sur la face supérieure 11 du dispositif de nettoyage 1. Ce dernier est équipé d'un couvercle 12 mobile entre deux positions, une position ouverte, dans laquelle l'espace de nettoyage 10 est accessible à un utilisateur, et une position fermée, dans laquelle le couvercle 12 délimite supérieurement l'espace de nettoyage 10. L'espace de nettoyage 10 est également délimité inférieurement par une platine 13 et latéralement par des bordures 14.

Avantageusement, l'espace de nettoyage 10 est pourvu d'au moins une cavité de nettoyage 3 de forme cylindrique ou conique. Avantageusement, la forme et les dimensions de la cavité de nettoyage 3 sont adaptées à accueillir toutes formes d'embouts intra-auriculaires 2. Dans la variante de réalisation illustrée, afin de nettoyer simultanément deux embouts intra-auriculaires 2, le dispositif de nettoyage 1 comporte deux cavités de nettoyage 3. Chaque cavité de nettoyage 3 est ménagée dans la platine 13 de l'espace de nettoyage 10.

Dans l'exemple illustré aux figures 1 et 2, la cavité de nettoyage 3 est conformée de manière à recevoir au centre de son fond 30 la partie distale 21 de l'embout intra-auriculaire 2. Ainsi, l'utilisateur insère chaque embout intra-auriculaire 2 dans une cavité de nettoyage 3, et dépose le contour d'oreille 23 si la prothèse en comporte un, sur la platine 14 de l'espace de nettoyage 10. Une fois que chaque prothèse auditive 20 est positionnée correctement, l'utilisateur ferme le couvercle 12 de l'espace de nettoyage 10 et lance le cycle de nettoyage en pressant un bouton d'activation du cycle de nettoyage.

Avantageusement, le dispositif de nettoyage 1 est adapté à réaliser un nettoyage de l'embout intra-auriculaire 2 par effleurement d'un vortex de liquide.

Conformément à l'invention, le dispositif de nettoyage 1 comporte une paroi double peau 31 présentant une peau interne 310, de section par exemple en « V », qui délimite latéralement la cavité de nettoyage 3, et dont l'extrémité supérieure est reliée à l'extrémité supérieure d'une peau externe 311, de section par exemple en « U ». En fait, tel qu'illustré à la figure 1, la peau interne 310 délimite, à sa base 313, une ouverture 317 surplombant, d'une part, le fond 30 de la cavité 3 qui est formé par une portion de la peau externe 311, et d'autre part, un orifice d'évacuation 32 de liquide de nettoyage que comporte la fond 30.

L'expression « l'ouverture 317 surplombant le fond 30 » signifie que l'ouverture 317 s'étend dans un plan positionné directement au dessus du fond 30 de la cavité de nettoyage 3.

La cavité 3 est conformée de manière à pouvoir accueillir tout embout intra-auriculaire 2 de forme quelconque, de manière telle que l'extrémité distale 21 de celui-ci émerge à travers l'ouverture 317 délimitée par la peau interne 310 située à une distance déterminée du fond 30 de la cavité 3.

Par ailleurs, une telle structure de la paroi double peau 31 a pour effet de ménager entre la peau interne 310 et la peau externe 311, un espace annulaire 312 qui ceinture la cavité de nettoyage 3 et qui communique avec cette dernière au niveau d'un espace de transit 314 s'étendant entre l'ouverture 317 délimitée par la base 313 de la peau interne 310 et le fond 30 de la cavité 3.

L'espace annulaire 312 est fermé supérieurement par une jonction 315 s'étendant entre les extrémités supérieures respectives des peaux interne 310 et externe 311.

Avantageusement, l'orifice d'évacuation 32 de liquide de nettoyage joue deux rôles, son premier rôle, consiste à évacuer le liquide de nettoyage présent dans la cavité de nettoyage 3, et son second rôle, consiste, de par sa position centrée dans le fond 30 de la cavité de nettoyage 3, à centrer la vortex de liquide au centre de la cavité de nettoyage 3.

Selon l'invention, ce dispositif de nettoyage 1 comporte encore des moyens d'injection 4 du liquide disposés dans l'espace annulaire 312 entre les deux peaux 310, 311 de la paroi double peau 31. L'injection du liquide de nettoyage au niveau de l'espace annulaire 312 évite toutes projections de liquide sur la partie proximale 22 de l'embout intra-auriculaire 2, cette partie proximale 22 étant protégée par la peau interne 310 de la double paroi 31.

Dans l'exemple illustré aux figures 1 et 2, les moyens d'injection 4 sont formés par une buse 40 d'injection, toutefois, les moyens d'injection 4, peuvent être formés par tous moyens permettant de projeter du liquide sous pression.

Dans l'exemple illustré aux figures 1 à 7, les moyens d'injection 4 sont adaptés à générer un vortex de liquide à l'intérieur de la cavité de nettoyage 3. A cet effet, les moyens d'injection 4 sont ménagés, de façon tangente, à la peau externe 311 projetant ainsi tangentiellement le liquide le long de cette dernière.

Dès lors, une fois projeté le long de la peau externe 311, grâce à la présence de l'espace annulaire 312, la flux de liquide est contraint d'entrer en rotation. De plus, le fond 30 présente une déclivité jusqu'à l'orifice d'évacuation 32. Cette déclivité du fond 30 dirige le flux de liquide en rotation vers l'orifice d'évacuation 32, le flux de liquide circulant alors depuis l'espace annulaire 312 vers le fond 30 de la cavité de nettoyage 3 en passant par l'espace de transit 314.

Avantageusement, la cinétique du flux de liquide en rotation, permet de retarder l'évacuation du liquide par l'orifice d'évacuation 32. De plus, l'alimentation en liquide est continue lors d'un cycle de nettoyage, le volume de liquide en rotation augmente et alimente le vortex dont le niveau s'élève à la fois dans la cavité de nettoyage 3 et dans l'espace annulaire 312. De cette manière, le vortex de liquide, dont l'œil est centré sur l'orifice d'évacuation 32, vient effleurer l'embout intra-auriculaire 2 et dissoudre le cérumen qui se trouve à sa surface. Afin de stabiliser et d'entretenir la vortex de liquide, le flux de liquide injecté dans chaque cavité de nettoyage 3 est continu et injecté sous pression constante tout au long du cycle de nettoyage.

Avantageusement, et comme illustré aux figures 1 et 2, le dispositif de nettoyage 1 comporte un orifice d'évacuation latéral 316 disposé dans l'espace annulaire 312. De préférence, l'orifice d'évacuation latéral 316 est ménagé dans la peau externe 311 à une hauteur seuil définie de manière à évacuer un flux trop important de liquide en circulation dans l'espace annulaire 312. L'orifice d'évacuation latéral 316 est disposé à une hauteur supérieure à celle des moyens d'injection 4. Il en résulte que cet orifice d'évacuation latéral 316 fait office de trop-plein, permettant de contrôler le flux de liquide en circulation dans l'espace annulaire 312 et par voie de faits de contrôler la hauteur du vortex de liquide dans la cavité de nettoyage 3. Cette caractéristique permet d'éviter que l'embout intra-auriculaire 2 ne soit submergé par le vortex de liquide.

Dans l'exemple illustré aux figures 1 à 3, le dispositif de nettoyage 1 comporte des moyens de réception 33 de la partie distale 21 de l'embout intra-auriculaire 2. Ces moyens de réception 33 s'étendent de manière saillante au-dessus de l'orifice d'évacuation 32 et permettent de surélever l'embout intra-auriculaire 2 de façon à ce qu'il n'entre pas en contact avec le fond 30 de la cavité de nettoyage 3. Ainsi, l'embout intra-auriculaire 2 ne contrevient pas à la circulation du flux de liquide et permet la formation du vortex de liquide. De préférence, les moyens de réception 33 sont formés par au moins une barre radiale 34. Toutefois, les moyens de réception 33 peuvent être formés par tous moyens mécaniques permettant de surélever l'embout intra-auriculaire 2 de façon à ce qu'il ne soit pas au contact de l'orifice d'évacuation 32. Ainsi, quelle que soit la forme de l'embout intra-auriculaire 2, sa partie distale 21 sera positionnée et maintenue au centre de la cavité de nettoyage 3 de la même manière.

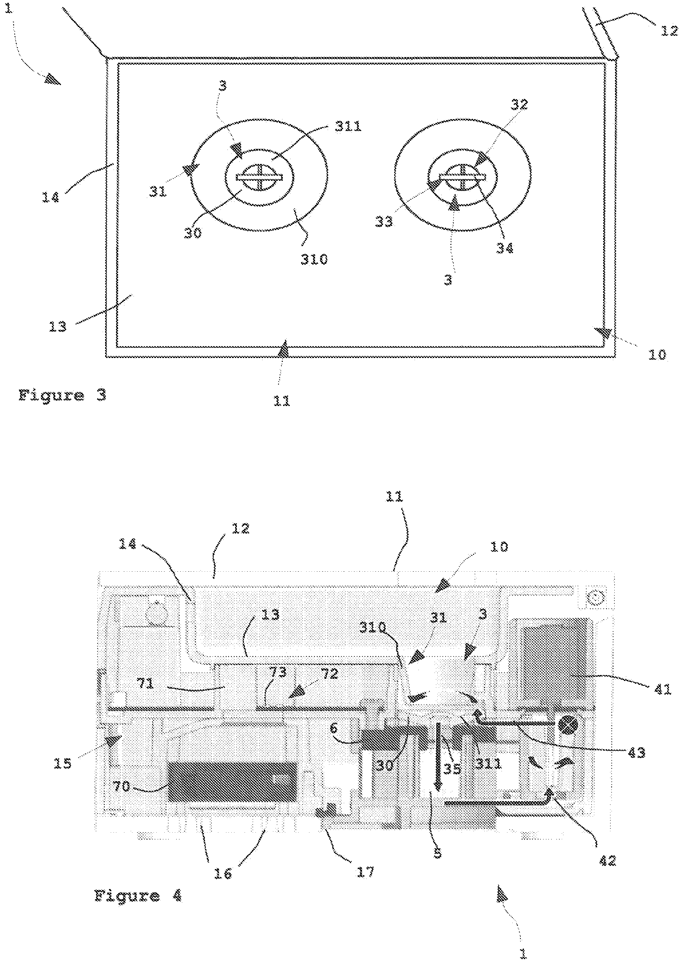

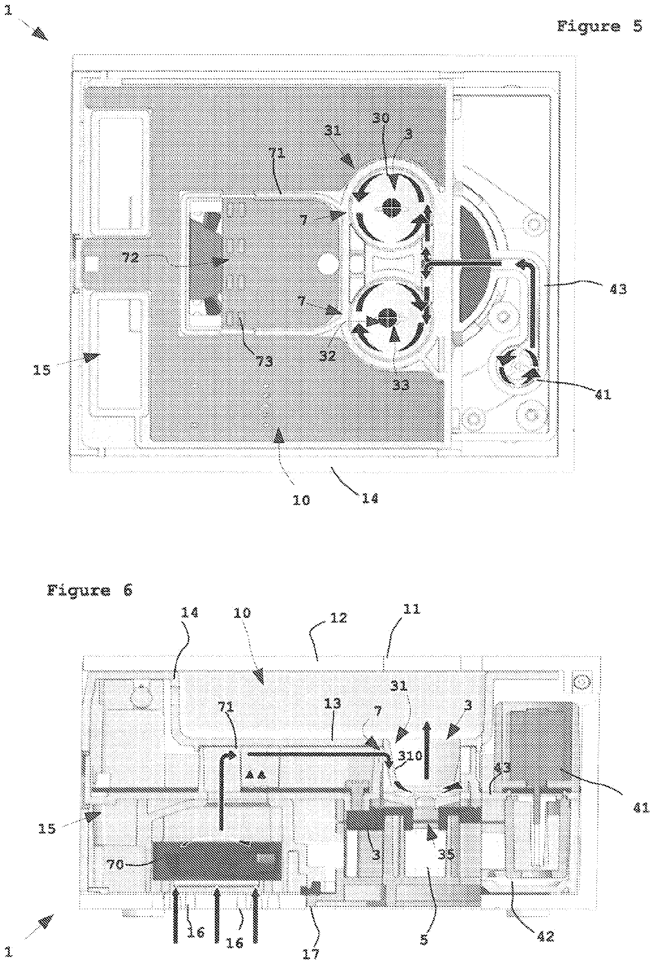

Comme illustré aux figures 4 et 6, le dispositif de nettoyage 1 comprend un réservoir 5 disposé de préférence dans l'espace interne 15 du dispositif de nettoyage 1 sous chaque cavité de nettoyage 3. Afin d'acheminer le liquide présent dans le réservoir 5, le dispositif de nettoyage 1 comporte un générateur 41 de flux de liquide. Le générateur 41 de flux de liquide est adapté, d'une part, à pomper la liquide stocké dans le réservoir 5 via un canal d'alimentation 42, et d'autre part, à projeter le liquide sous pression constante, au travers d'un canal d'injection 43, vers les moyens d'injection 4 injectant le liquide dans la cavité de nettoyage 3 en continu à pression constante. Le canal d'injection 43 alimente les moyens d'injection 4 des deux cavités de nettoyage 3 que comporte le dispositif de nettoyage 1.

Le réservoir 5 communiqué également avec chaque cavité de nettoyage 3 du dispositif de nettoyage 1 par l'intermédiaire de l'orifice d'évacuation 32 relié à un canal d'évacuation 35 communiquant avec le réservoir 5. Ainsi, au cours du cycle de nettoyage, le flux de liquide évacué par l'orifice d'évacuation 32 suit le canal d'évacuation 35 pour rejoindre le réservoir 5 (illustré figure 4). Afin d'éviter que le cérumen détaché de l'embout intra-auriculaire 2, ne tombe dans le réservoir 5, un filtre est disposé au sein du canal d'évacuation 35.

Comme illustré aux figures 4 et 5, lors d'un cycle de nettoyage, la liquide est utilisé en circuit fermé, c'est-à-dire, que la générateur de flux de liquide 41 génère et injecte en continue à pression constante un flux de liquide dans chaque cavité de nettoyage 3. Le flux de liquide injecté dans chaque cavité de nettoyage 3 génère un vortex avant de retomber dans le réservoir 5 via l'orifice d'évacuation 32, alors que le vortex est entretenu par l'injection constante de flux de liquide. Une fois dans le réservoir 5, la liquide est de nouveau injecté dans chaque cavité de nettoyage 3 par le générateur de flux de liquide 41. La circulation du flux de liquide au sein du dispositif de nettoyage 1 lors d'un cycle de nettoyage est schématisée aux figures 4 et 5 par des flèches pleines.

Il est à noter, que l'orifice d'évacuation latéral 316 communique avec le réservoir 5 par un conduit 318 aménagé à l'extérieur de la peau externe 311 de la paroi double peau 31.

Avantageusement, le dispositif de nettoyage 1 comprend un bouchon 6 situé entre la cavité de nettoyage 3 et le réservoir 5, et dans lequel est aménagé le canal d'évacuation 35. Ce bouchon 6 est mobile entre trois positions, une première position ouverte, dans laquelle le canal d'évacuation 35 est disposé dans le prolongement axial de l'orifice d'évacuation 32 et du réservoir 5, le liquide pouvant ainsi circuler depuis chaque cavité de nettoyage 3 vers le réservoir 5, une deuxième position formée, dans laquelle le liquide est cantonné dans le réservoir 5, et une troisième position de vidange, dans laquelle le liquide peut être vidangé depuis le réservoir 5 vers l'extérieur du dispositif de nettoyage 1, la vidange est effectuée par gravité au travers d'un siphon 50 obstrué par le bouchon 6. Le bouchon 6 assure un usage facile et pratique du dispositif de nettoyage 1. Ainsi, lorsque le bouchon 6 est en position fermée, le dispositif de nettoyage 1 peut être transporté, le réservoir 5 plein, sans risque de fuites de liquide lorsqu'il n'est pas maintenu horizontalement.

Une fois le cycle de nettoyage terminé, afin de sécher rapidement un embout intra-auriculaire 2, le dispositif de nettoyage 1 comporte des moyens de projection d'air 7 disposés dans l'espace annulaire 312 permettant de projeter de l'air tourbillonnant dans chaque cavité de nettoyage 3. à ces fins, les moyens de projection d'air 7 sont ménagés dans la peau externe 311 sous la jonction 315 entre les peaux interne 310 et externe 311 de la double paroi 31. Ainsi, le flux d'air projeté entre en rotation au sein de l'espace annulaire 312. Le flux d'air tourbillonnant s'échappe de l'espace annulaire 312 par la cavité de nettoyage 3 en passant par l'espace de transit 314. Cette caractéristique permet d'éliminer le liquide résiduel qui reste englué à la surface de l'embout intra-auriculaire 2.

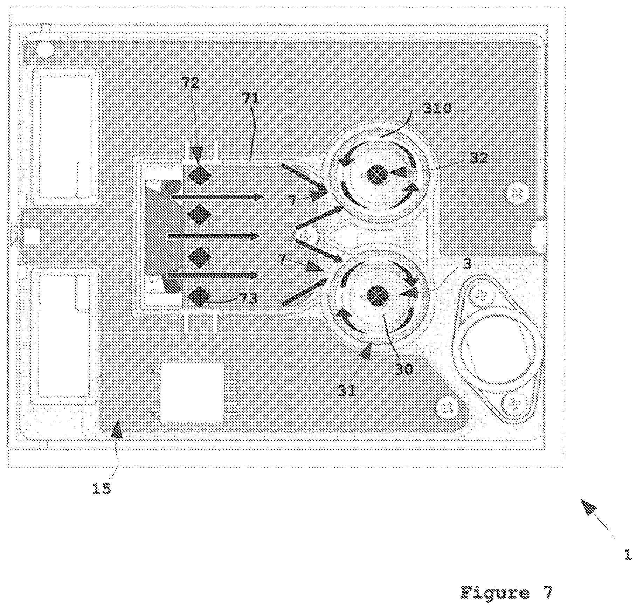

Afin de générer un flux d'air alimentant les moyens de projection d'air 7, le dispositif de nettoyage 1 comporte dans son espace interne 15 un générateur de flux d'air 70 adapté à aspirer l'air ambiant par l'intermédiaire de pores 16 communiquant avec l'extérieur du dispositif de nettoyage 1. De préférence, les pores 16 sont aménagés dans la face inférieure 17 du dispositif de nettoyage 1. Le générateur de flux d'air 70 projette ensuite un flux d'air au travers d'un conduit d'aération 71 acheminant le flux d'air jusqu'aux moyens de projection d'air 7. De préférence, le conduit d'aération 71 présente une section en « L » de façon à guider le plus directement possible le flux d'air vers les moyens de projection 7, préservant ainsi l'intensité du flux d'air et l'efficacité du séchage de l'embout intra-auriculaire 2. Le cheminement du flux d'air lors du séchage d'un embout intra-auriculaire 2, depuis le générateur de flux d'air 70 vers la cavité de nettoyage 3, est schématisé par des flèches pleines aux figures 6 et 7.

Avantageusement, selon une caractéristique additionnelle de l'invention illustrée aux figures 6 et 7, le dispositif de nettoyage 1 comprend des moyens de chauffage 72 du flux d'air. De préférence, ces moyens de chauffage se situent dans le conduit d'aération 71 en amont des moyens de projection d'air 7. Le fait de chauffer le flux d'air, permet d'éliminer plus rapidement, par évaporation, le liquide résiduel qui se trouve à la surface de l'embout intra-auriculaire 2 après un cycle de nettoyage. Dans cet exemple, les moyens de chauffage 72 du flux d'air sont formés par des résistances électriques 73, toutefois, les moyens de chauffage 72 du flux d'air peuvent être formés par tous moyens électriques, électroniques, mécaniques, thermiques, pouvant chauffer un flux d'air. Le chauffage du flux d'air est schématisé par des triangles pleins à la figure 6 et des losanges pleins à la figure 7.

Il est à noter que dans une configuration spécifique du dispositif de nettoyage 1, les moyens de projection d'air 7 peuvent fonctionner au cours du cycle de nettoyage et en même temps que les moyens d'injection 4 de liquide. En effet, paramétré de façon adéquate, le générateur de flux d'air 70 est tout à fait capable de générer un flux d'air adapté à participer au contrôle du vortex de liquide nettoyant l'embout intra-auriculaire 2.

Selon une caractéristique additionnelle de l'invention illustrée aux figures 4 et 6, en plus des propriétés désinfectantes du liquide de nettoyage, l'espace de nettoyage 10 est équipé d'une lampe UVC 8 adaptée à diffuser au sein de l'espace de nettoyage 10 et de la cavité de nettoyage 3, des ondes UVC. Ainsi, une prothèse auditive 20 disposée dans l'espace de nettoyage 10 lorsque le couvercle 12 est fermé, sera désinfectée par irradiation UVC. Cette caractéristique permet de terminer chaque cycle de nettoyage, une fois l'embout 2 séché, par une désinfection de tous les éléments de la prothèse auditive 20 (embout intra-auriculaire 2 et éventuellement le contour d'oreille 23 si la prothèse en comporte un). L'irradiation UVC de la prothèse auditive 20 a pour avantage d'éliminer des éléments irritants ou allergènes toujours présents à la suite du cycle de nettoyage, et ainsi de diminuer les irritations du conduit auditif et du contour de l'oreille de l'utilisateur de la prothèse auditive 20.

L'utilisation d'un flux d'air chauffé pour éliminer, par évaporation, le liquide résiduel qui se trouve à la surface de l'embout intra-auriculaire 2, entraine une déperdition de la quantité de liquide présent dans le réservoir 5. Afin d'éviter que l'utilisateur ne lance un cycle de nettoyage alors que la quantité de liquide présente dans le réservoir 5 est insuffisante, le réservoir 5 est équipé de moyens de détection du niveau de liquide. Ces moyens de détection peuvent être constitués par des capteurs résistifs ou tous autres moyens mécaniques ou électroniques capable de se déclencher lorsque le niveau de liquide descend en dessous d'un niveau seuil prédéfini. Le dispositif de nettoyage 1 comporte également des moyens d'avertissement (par exemple un signal sonore, un signal visuel sur un écran etc.) adaptés à signaler à l'utilisateur qu'il doit vidanger et remplir à nouveau le réservoir 5.

De plus, le liquide de nettoyage doit être changé au bout d'un nombre défini de cycles de nettoyage, ou bien lorsque le niveau de liquide présent dans le réservoir devient insuffisant, par exemple suite à des pertes de liquide par évaporation.

Dans l'objectif de remplir le réservoir 5 disposé au sein de l'espace interne 15 du dispositif de nettoyage 1, la demanderesse a développé une unité de rechargement 9 de structure simple et peu coûteuse.

Cette unité de rechargement 9 permet avantageusement de procéder à un remplissage facile, propre, et sans éclaboussures du réservoir 5 du dispositif de nettoyage 1.

à ces fins, au moins une cavité de nettoyage 3 est utilisée comme interface pour faire transiter le liquide de nettoyage entre l'unité de rechargement 9 le réservoir 5.

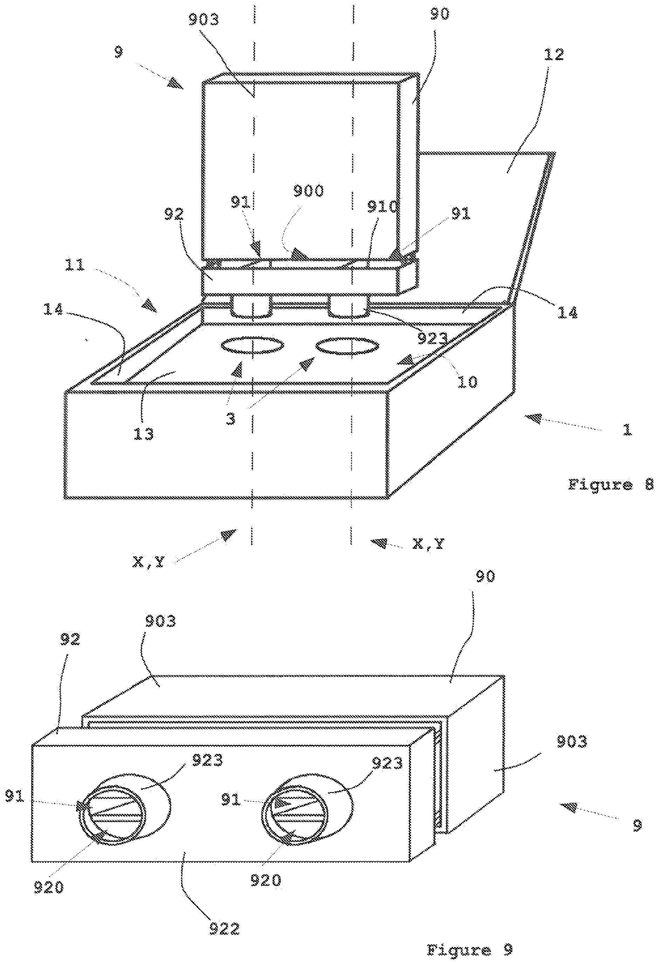

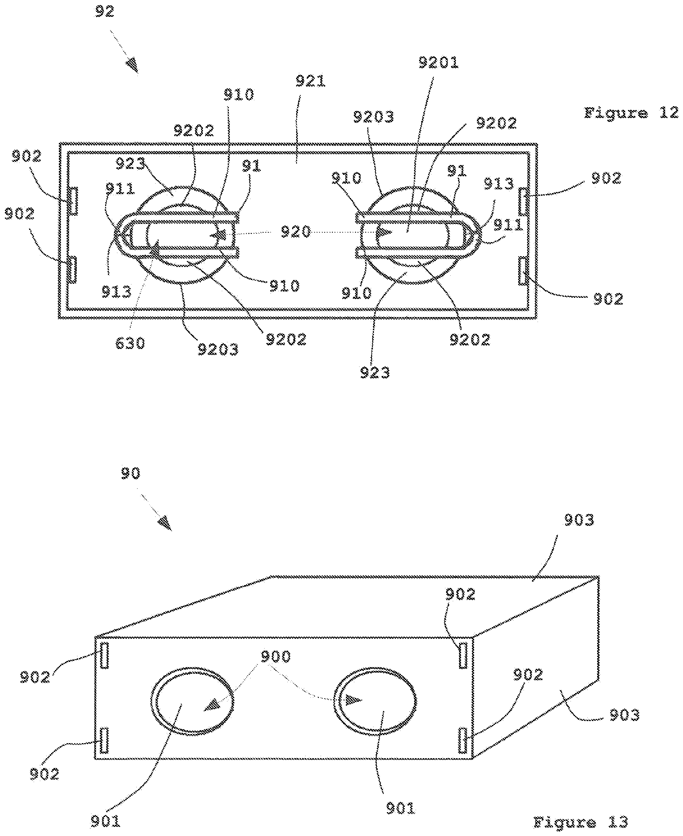

Dans l'exemple illustré aux figures 8 à 10 et 13, l'unité de rechargement 9 comporte une cuve 90 en forme de pavé. Bien entendu, d'autres formes peuvent également être envisagées telles que cubique, sphérique, cylindrique etc. En référence à la figure 13, la cuve 90 comporte sur une de ses faces deux ouvertures 900 disposées de manière telle qu'elles peuvent être situées chacune dans l'axe X de la cavité de nettoyage 3 du dispositif de nettoyage 1 lorsque l'unité de rechargement 9 est située vis à vis de l'espace de nettoyage 10.

Afin de conserver, de façon hermétique, le liquide de nettoyage à l'intérieur de la cuve 90, les ouvertures 900 sont fermées chacune par un opercule 901 imperméable qui retient le liquide à l'intérieur de la cuve 90 aussi longtemps qu'il n'est ni pelé ni perforé.

Dans le présent exemple, l'opercule 901 est susceptible d'être perforé par un perforateur 91 afin de libérer le liquide de la cuve 90 et permettre son écoulement à travers les ouvertures 900. I1 est à noter que la cuve 90 pourrait également contenir une poche hermétique remplie de liquide de nettoyage et dont une portion remplirait une fonction d'opercule obturant les ouvertures 900. De la même manière, une telle poche serait par conséquent susceptible d'être perforée par un perforateur 91 afin de libérer la liquide et de permettre son écoulement à travers les ouvertures 900.

Comme illustré aux figures 8 à 12, l'unité de rechargement 9 est en outre équipée d'un capuchon 92, recouvrant les ouvertures 900, pourvu de deux canaux 920 s'étendant chacun dans l'axe Y d'une ouverture 900. Le capuchon 92 constitue en fait une interface de connexion entre l'unité de rechargement 9 et le dispositif de nettoyage 1 à recharger. Il comporte une face interne 921 disposée face aux ouvertures 900 de la cuve 90 et une face externe 922 destinée à être placée au contact du dispositif de nettoyage 1, lors du rechargement de ce dernier.

Aux fins de libération du liquide contenu dans la cuve 90 de l'unité de rechargement 9, la capuchon 92 comporte deux perforateurs 91 adaptés à perforer chacun un opercule 901 et laisser s'écouler le liquide une fois l'opercule 901 perforé.

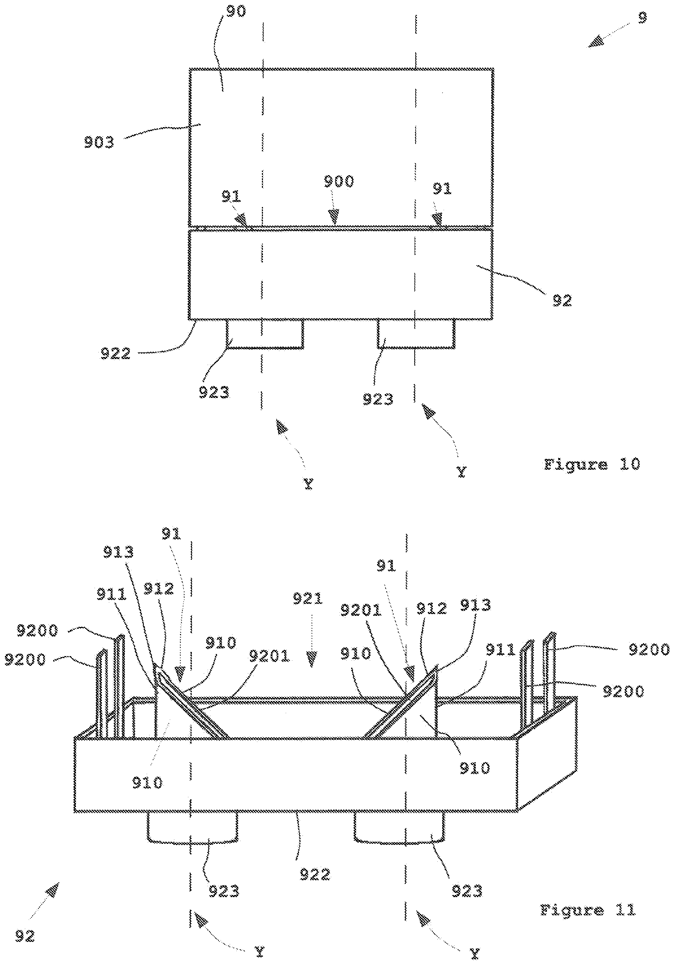

En fait, chaque perforateur 91 s'étend depuis la face interne 921 du capuchon 92 en direction des ouvertures 900 de la cuve 90. De plus, le capuchon 92 est mobile par rapport à la cuve 90 entre une position préliminaire (illustrée aux figures 8 et 9) dans laquelle les perforateurs 91 sont écartés de chaque ouverture 900, et une position de percussion (illustrée à la figure 10) dans laquelle chaque perforateur 91 s'étend dans une ouverture 900 à travers l'opercule 901 ainsi perforé. La position préliminaire correspond à la position dans laquelle se trouve le capuchon 92 avant l'utilisation de l'unité de rechargement 9, tandis que la position de percussion est atteinte lors de l'utilisation de l'unité de rechargement 9.

Dans cet exemple, le capuchon 92 comporte deux paires de rails 9200 s'étendant chacun parallèlement à l'axe Y d'une ouverture 900. Ces rails 9200 sont prévus aptes à coopérer avec des coulisses 902 ménagées de manière appropriée sur la paroi 903 de la cuve 90. Ces caractéristiques permettent au capuchon 92 d'être mobile en translation par rapport à la cuve 90 selon une direction parallèle à l'axe Y d'une ouverture 900.

Chaque rail 9200 coopère avec une coulisse 902 de manière à ce que le capuchon 92 se déplace entre ses deux positions. Ainsi, le capuchon 92 peut à la fois remplir un rôle de protecteur d'un opercule 900 lorsqu'il est en position préliminaire et que l'unité de rechargement 9 doit être conservée intacte en attendant son utilisation, et un rôle de perforateur d'un opercule 900 lorsqu'il est en position de percussion, tel qu'il sera décrit ci-dessous.

Par ailleurs, les perforateurs 91 du capuchon 92 sont avantageusement conformés de manière à définir chacun une canule d'écoulement du liquide de nettoyage contenu dans la cuve 90 au travers de chaque ouverture 900 de la cuve 90 et de chaque canal 920 du capuchon 92. à cet effet, chaque perforateur 91 présente deux faces latérales 910 reliées entre elles par une base 911 et s'étend dans le sens de déplacement du capuchon 92, dans un plan perpendiculaire à la face interne 921 du capuchon 92. Le bord libre 912 de la base 911 de chaque perforateur 91 est en outre conformé de manière à définir une pointe perforatrice 913, faisant saillie au niveau de la base 911, et apte à perforer un opercule 900. Par ailleurs, de par sa structure et son positionnement transversalement à un canal 920, chaque perforateur 92 compartimente ce dernier en trois zones, une zone centrale 9201 de canalisation du liquide, située entre ses deux faces latérales 9202, et deux zones latérales 9202 situées entre chaque face latérale 910 et le bord 9203 du canal 920. Il est à noter qu'un perforateur 91 peut présenter différentes formes dès lors qu'il s'étend depuis la face interne 921 du capuchon 92 en direction des ouvertures 900.

Ainsi, lorsque le capuchon 92 passe de sa position préliminaire à sa position de percussion, la pointe perforatrice 913 du perforateur 91 entre en contact avec un opercule 900, le perfore, l'enroule et libère le liquide contenu dans la cuve 90. Avantageusement, chaque zone latérale 9202 réalise un appel d'air permettant au liquide de s'écouler au travers de la zone centrale 9201.

Afin d'éviter tous déplacements non désirés du capuchon 92 depuis sa position préliminaire vers sa position de percussion, des moyens de blocage sont disposés à la jonction entre le capuchon 92 et la cuve 90. Ces moyens de blocage sont prévus amovibles, et permettent au capuchon 92 de rester en position préliminaire jusqu'à l'utilisation de l'unité de rechargement 9. Les moyens de blocage peuvent par exemple être définis par une lanière en plastique disposée à la jonction entre un opercule 900 et la cuve 90, cette lanière bloquant le déplacement du capuchon 92.

Dans l'exemple illustré aux figures 8 à 12, le capuchon 92 comporte par ailleurs deux manchons 923 s'étendant sur sa face externe 922 et définissant le canal 920. Chaque manchon 923 est conformé de façon complémentaire à la cavité de nettoyage 3 du dispositif de nettoyage 1. Ainsi, les manchons 923 constituent des moyens de connexion entre l'unité de rechargement 9 et le dispositif de nettoyage 1.

Lorsque les moyens d'avertissement du dispositif de nettoyage 1 indiquent à l'utilisateur qu'il est nécessaire de vidanger et de remplir à nouveau le réservoir 5 de liquide de nettoyage, l'utilisateur, l'utilisateur fait passer le bouchon 6 dans sa position de vidange afin d'effectuer la vidange du réservoir 5. Suite à la vidange, l'utilisateur amène le bouchon 6 dans sa position ouverte dans laquelle le canal d'évacuation 35 et l'orifice d'évacuation 32 se trouvent disposés dans le prolongement axial du réservoir 5, il est alors possible de remplir ce dernier à l'aide de l'unité de rechargement 9 par gravité en utilisant les cavités de nettoyage 3 du dispositif de nettoyage 1 comme interface avec le réservoir 5.

Afin de procéder au rechargement du réservoir 5 du dispositif de nettoyage 1, il convient de retirer les moyens de blocage du déplacement du capuchon 92, avant que chaque manchon 923 du capuchon 92 ne soit positionné au sein d'une cavité de nettoyage 3 de manière à ce que l'unité de rechargement 9 s'emboîte avec le dispositif de nettoyage 1. Ainsi, chaque ouverture 900 de la cuve 90 est alignée avec un canal 920 du capuchon 92 un orifice d'évacuation du liquide 32 lui même aligné axialement avec un canal d'évacuation 35 donnant accès au réservoir 5.

Pour finir, l'utilisateur applique une pression verticale sur la cuve 90 de l'unité de rechargement 9. Sous l'effet de cette pression, le capuchon 92 se déplace, depuis sa position préliminaire vers sa position de percussion. Chaque perforateur 91 est alors apte à entrer en contact avec un opercule 901, perce celui-ci et contrôle, de par sa disposition et sa conformation, l'écoulement du liquide de nettoyage contenu dans la cuve 90 de l'unité de rechargement 9 vers le réservoir 5 du dispositif de nettoyage 1. Ainsi, l'écoulement du liquide de nettoyage est réalisé par gravitation.

Dans le présent exemple, la cavité de nettoyage du dispositif de nettoyage 1 rempli deux fonctions, une fonction de nettoyage d'une prothèse auditive (20) et de son embout (2) et une fonction de remplissage au réservoir 5.

Grâce à l'invention et à la complémentarité entre l'unité de rechargement 9 et le dispositif de nettoyage 1, il est ainsi possible de réaliser un remplissage propre du réservoir 5, sans éclaboussures et sans perte de liquide.

## Revendications

1. Dispositif de nettoyage (1) d'une prothèse auditive (20) par effleurement d'un liquide, la prothèse auditive (20) comprenant un embout (2) intra-auriculaire, le dispositif de nettoyage (1) comprenant une cavité de nettoyage (3) adaptée à recevoir l'embout (2) et qui est équipée en son fond (30) d'un orifice d'évacuation (32) du liquide, la cavité de nettoyage (3) étant délimitée latéralement par une peau interne (310) ceinturée par une peau externe (311), la peau interne (310) et la peau externe (311) formant entre elles un espace annulaire (312) fermé supérieurement, l'espace annulaire (312) ceinturant la cavité de nettoyage (3),
la peau externe (311) comportant des moyens d'injection (4) du liquide disposés dans l'espace annulaire (312) et configurés pour injecter le liquide dans l'espace annulaire (312), la peau interne (310) délimitant à sa base (313) une ouverture (317) surplombant le fond (30) et permettant le passage du liquide vers l'intérieur de la cavité de nettoyage (3),
l'ouverture (317) et le fond (30) de la cavité de nettoyage (3) délimitant entre eux un espace de transit (314), l'espace annulaire (312) étant en communication fluidique avec la cavité de nettoyage (3) au niveau dudit espace de transit (314).

2. Dispositif de nettoyage (1) selon la revendication 1, **caractérisé en ce que** la peau externe (310) comporte à un niveau supérieur aux moyens d'injection (4) un orifice d'évacuation latéral (316) permettant d'évacuer un excès de liquide circulant entre les peaux interne (310) et externe (311).

3. Dispositif de nettoyage (1) selon l'une des revendications 1 et 2, **caractérisé en ce que** les moyens d'injection (4) sont tangents à la peau externe (311) de façon à projeter tangentiellement le liquide le long de la peau externe (311).

4. Dispositif de nettoyage (1) selon l'une des revendications 1 à 3, **caractérisé en ce qu'**il comprend un réservoir (5) équipé d'un bouchon (6) qui est mobile entre une position fermée dans laquelle le liquide est cantonné dans le réservoir (5), une position ouverte dans laquelle le liquide circule entre la cavité de nettoyage (3) et le réservoir (5), et une position de vidange dans laquelle le liquide est vidangé depuis le réservoir (5) hors du dispositif de nettoyage (1).

5. Dispositif de nettoyage selon la revendication 4, **caractérisé en ce que** le réservoir (5) comporte des moyens de détection du niveau du liquide, ces moyens de détection étant adaptés à déclencher l'émission d'un signal d'avertissement lorsque le niveau de liquide, présent à l'intérieur du réservoir, atteint un niveau seuil prédéfini.

6. Dispositif de nettoyage (1) selon l'une des revendications 1 à 5, **caractérisé en ce que** le fond (30) comprend des moyens de réception (33) saillants et adaptés à recevoir l'embout (2) de façon surélevée par rapport au fond (30).

7. Dispositif de nettoyage (1) selon l'une des revendications 1 à 6, **caractérisé en ce que** la peau externe (311) comprend des moyens de projection (7) d'air dans l'espace annulaire (312).

8. Dispositif de nettoyage (1) selon la revendication 7, **caractérisé en ce qu'**il comprend des moyens de chauffage (72) chauffant le flux d'air projeté.

9. Dispositif de nettoyage (1) selon l'une des revendications 1 à 8, **caractérisé en ce qu'**il comprend une lampe UVC (8) adaptée à diffuser des ondes UVC en direction de la prothèse auditive (20) de manière à réaliser une désinfection par irradiation UVC.

10. Ensemble comprenant un dispositif de nettoyage (1) selon l'une des revendications 1 à 9 et une unité de rechargement (9) de liquide de nettoyage adaptée à remplir un réservoir (5) du dispositif de nettoyage (1), **caractérisé en ce que** l'unité de rechargement (9) comporte un manchon (923) adapté pour coopérer avec au moins une cavité de nettoyage (3) du dispositif de nettoyage (1), de manière à transférer le liquide de nettoyage depuis l'unité de rechargement (9) vers le réservoir (5) du dispositif de nettoyage (1).

11. Ensemble selon la revendication 10, **caractérisé en ce que** l'unité de rechargement (9) comporte, d'une part, une cuve (90) équipée d'une ouverture (900) fermée par un opercule (901), et d'autre part, des moyens de connexion qui sont adaptés à perforer l'opercule (901) lorsqu'ils sont mis en connexion avec une cavité de nettoyage (3) du dispositif de nettoyage (1).

12. Ensemble selon l'une des revendications 10 et 11, **caractérisé en ce que** l'unité de rechargement (9) comporte un capuchon (92) qui comprend un canal (920), un perforateur (91) adapté à perforer l'opercule (901) et des moyens de connexion avec la cavité de nettoyage (3), le capuchon (92) étant mobile par rapport à la cuve (90) entre une position préliminaire dans laquelle le perforateur (91) est écarté de l'ouverture (900), et une position de percussion dans laquelle le perforateur (91) perfore l'opercule (900) et libère le liquide par le canal (920) .

13. Ensemble selon l'une des revendications 11 et 12, **caractérisé en ce que** le perforateur (91) définit une canule d'écoulement du liquide de nettoyage contenu dans la cuve (90) au travers du canal (920) du capuchon (92) jusqu'à la cavité de nettoyage (3).

14. Ensemble selon l'une des revendications 11 à 13, **caractérisé en ce que** le perforateur (91) s'étend dans le sens de déplacement du capuchon (92) et présente deux faces latérales (910) reliées entre elles par une base (911) comportant une pointe perforatrice (913) apte à perforer l'opercule (901).

## Patentansprüche

1. Reinigungsvorrichtung (1) für ein Hörgerät (20) durch Kontakt mit einer Flüssigkeit, das Hörgerät (20) umfassend einen In-Ear-Stöpsel (2), die Reinigungsvorrichtung (1) umfassend einen Reinigungshohlraum (3), der angepasst ist, um den Stöpsel (2) aufzunehmen, und der an seinem Boden (30) mit einer Öffnung (32) zum Ablassen der Flüssigkeit ausgestattet ist, wobei der Reinigungshohlraum (3) durch eine innere Schicht (310), die von einer äußeren Schicht (311) umgeben ist, seitlich begrenzt ist, wobei die innere Schicht (310) und die äußere Schicht (311) miteinander einen oben geschlossenen Ringraum (312) ausbilden, wobei der Ringraum (312) den Reinigungshohlraum (3) umgibt,
wobei die äußere Schicht (311) Mittel (4) zum Einspritzen der Flüssigkeit, die in dem Ringraum (312) angeordnet sind, aufweist und konfiguriert ist, um die Flüssigkeit in den Ringraum (312) einzuspritzen, wobei die innere Schicht (310) an ihrer Basis (313) eine Mündung (317) begrenzt, die den Boden (30) überragt und den Durchlauf der Flüssigkeit nach innen in den Reinigungshohlraum (3) ermöglicht,
wobei die Mündung (317) und der Boden (30) des Reinigungshohlraums (3) untereinander einen Durchgangsraum (314) begrenzen, wobei der Ringraum (312) in Fluidverbindung mit dem Reinigungshohlraum (3) an dem Transitraum (314) steht.

2. Reinigungsvorrichtung (1) nach Anspruch 1, **dadurch gekennzeichnet, dass** die äußere Schicht (310) über den Einspritzmitteln (4) eine seitliche Ablassöffnung (316) aufweist, die das Ablassen überschüssiger Flüssigkeit, die zwischen der inneren Schicht (310) und der äußeren Schicht (311) zirkuliert, ermöglicht.

3. Reinigungsvorrichtung (1) nach einem der Ansprüche 1 und 2, **dadurch gekennzeichnet, dass** die Einspritzmittel (4) an der äußeren Schicht (311) anliegen, um die Flüssigkeit entlang der äußeren Schicht (311) tangential zu verspritzen.

4. Reinigungsvorrichtung (1) nach einem der Ansprüche 1 bis 3, **dadurch gekennzeichnet, dass** sie einen Behälter (5) umfasst, der mit einem Stopfen (6) ausgestattet ist, der zwischen einer geschlossenen Position, in der die Flüssigkeit in dem Behälter (5) untergebracht ist, einer offenen Position, in der die Flüssigkeit zwischen dem Reinigungshohlraum (3) und dem Behälter (5) zirkuliert, und einer Entleerungsposition, in der die Flüssigkeit aus dem Behälter (5) von der Reinigungsvorrichtung (1) entleert wird, bewegbar ist.

5. Reinigungsvorrichtung nach Anspruch 4, **dadurch gekennzeichnet, dass** der Behälter (5) Mittel zum Erfassen des Flüssigkeitsstands aufweist, wobei diese Erfassungsmittel angepasst sind, um die Aussendung eines Warnsignals auszulösen, wenn der Flüssigkeitsstand, der innerhalb des Behälters vorhanden ist, einen vordefinierten Schwellenwert erreicht.

6. Reinigungsvorrichtung (1) nach einem der Ansprüche 1 bis 5, **dadurch gekennzeichnet, dass** der Boden (30) vorstehende Aufnahmemittel (33) umfasst und angepasst ist, um den Stöpsel (2) relativ zu dem Boden (30) hochliegend aufzunehmen.

7. Reinigungsvorrichtung (1) nach einem der Ansprüche 1 bis 6, **dadurch gekennzeichnet, dass** die äußere Schicht (311) Mittel (7) zum Blasen von Luft in den Ringraum (312) umfasst.

8. Reinigungsvorrichtung (1) nach Anspruch 7, **dadurch gekennzeichnet, dass** sie Heizmittel (72) umfasst, die den geblasenen Luftstrom erhitzen.

9. Reinigungsvorrichtung (1) nach einem der Ansprüche 1 bis 8, **dadurch gekennzeichnet, dass** sie eine UVC-Lampe (8) umfasst, die angepasst ist, um UVC-Wellen in Richtung des Hörgeräts (20) auszubreiten, um eine Desinfektion durch UVC-Bestrahlung durchzuführen.

10. Anordnung, umfassend eine Reinigungsvorrichtung (1) nach einem der Ansprüche 1 bis 9 und eine Einheit (9) zum Nachfüllen von Reinigungsflüssigkeit, die angepasst ist, um einen Behälter (5) der Reinigungsvorrichtung (1) aufzufüllen, **dadurch gekennzeichnet, dass** die Nachfülleinheit (9) eine Hülse (923) aufweist, die angepasst ist, um mit mindestens einem Reinigungshohlraum (3) der Reinigungsvorrichtung (1) zusammenzuwirken, um die Reinigungsflüssigkeit aus der Nachfülleinheit (9) in den Behälter (5) der Reinigungsvorrichtung (1) zu übertragen.

11. Anordnung nach Anspruch 10, **dadurch gekennzeichnet, dass** die Nachfülleinheit (9) einerseits eine Wanne (90), die mit einer Mündung (900) ausgestattet ist, die durch ein Verschlussorgan (901) geschlossen ist, und andererseits Verbindungsmittel, die angepasst sind, um das Verschlussorgan (901) zu perforieren, wenn sie mit einem Reinigungshohlraum (3) der Reinigungsvorrichtung (1) verbunden werden, aufweist.

12. Anordnung nach einem der Ansprüche 10 und 11, **dadurch gekennzeichnet, dass** die Nachfülleinheit (9) eine Kappe (92), die einen Kanal (920) umfasst, eine Lochvorrichtung (91), die zum Perforieren des Verschlussorgans (901) angepasst ist, und Mittel zum Verbinden mit dem Reinigungshohlraum (3) aufweist, wobei die Kappe (92) in Bezug auf die Wanne (90) zwischen einer vorläufigen Position, in der die Lochvorrichtung (91) von der Mündung (900) entfernt ist, und einer Schlagposition, in der die Lochvorrichtung (91) das Verschlussorgan (900) perforiert und die Flüssigkeit durch den Kanal (920) freisetzt, bewegbar ist.

13. Anordnung nach einem der Ansprüche 11 und 12, **dadurch gekennzeichnet, dass** die Lochvorrichtung (91) eine Strömungskanüle der Reinigungsflüssigkeit, die in der Wanne (90) enthalten ist, durch den Kanal (920) der Kappe (92) zu dem Reinigungshohlraum (3) definiert.

14. Anordnung nach einem der Ansprüche 11 bis 13, **dadurch gekennzeichnet, dass** sich die Lochvorrichtung (91) in die Bewegungsrichtung der Kappe (92) erstreckt und zwei Seitenflächen (910) vorweist, die durch eine Basis (911), die eine perforierende Spitze (913) aufweist, die geeignet ist, um das Verschlussorgan (901) zu perforieren, miteinander verbunden sind.

## Claims

1. A device (1) for cleaning a hearing aid (20) by light touching with a liquid, the hearing aid (20) comprising an earmold (2), the cleaning device (1) comprising a cleaning cavity (3) suitable for receiving the earmold (2), and the bottom (30) of the cavity is provided with a liquid discharge port (32), the cleaning cavity (3) being laterally delimited by an inner skin (310) surrounded by an outer skin (311), the inner skin (310) and the outer skin (311) forming an annular space (312) therebetween which is closed at the top, the annular space (312) surrounding the cleaning cavity (3),
the outer skin (311) having means (4) for injecting the liquid which are arranged in the annular space (312) and configured for injecting the liquid into the annular space (312), the inner skin (310) delimiting at its base (313) an opening (317) overhanging the bottom (30) and allowing the liquid to pass into the cleaning cavity (3),
the opening (317) and the bottom (30) of the cleaning cavity (3) delimiting a transit space (314) therebetween, the annular space (312) being in fluid communication with the cleaning cavity (3) at said transit space (314).

2. The cleaning device (1) according to claim 1, **characterized in that** the outer skin (310) comprises, at a level higher than the injection means (4), a lateral discharge port (316) making it possible to discharge excess liquid flowing between the inner skin (310) and outer skin (311).

3. The cleaning device (1) according to any of claims 1 and 2,
**characterized in that** the injection means (4) are tangential to the outer skin (311) so as to spray the liquid tangentially along the outer skin (311).

4. The cleaning device (1) according to any of claims 1 to 3,
**characterized in that** it comprises a reservoir (5) provided with a cap (6) which is movable between a closed position in which the liquid is confined in the reservoir (5), an open position in which the liquid flows between the cleaning cavity (3) and the reservoir (5), and an emptying position in which the liquid is emptied from the reservoir (5) out of the cleaning device (1).

5. The cleaning device according to claim 4, **characterized in that** the reservoir (5) comprises means for detecting the liquid level, said detection means being suitable for triggering the emission of a warning signal when the level of liquid present inside the reservoir reaches a predefined threshold level.

6. The cleaning device (1) according to any of claims 1 to 5,
**characterized in that** the bottom (30) comprises projecting receiving means (33) suitable for receiving the earmold (2) in a raised manner relative to the bottom (30).

7. The cleaning device (1) according to any of claims 1 to 6,
**characterized in that** the outer skin (311) comprises means (7) for projecting air into the annular space (312).

8. The cleaning device (1) according to claim 7, **characterized in that** it comprises means (72) for heating the projected flow of air.

9. The cleaning device (1) according to any of claims 1 to 8,
**characterized in that** it comprises a UVC lamp (8) suitable for emitting UVC waves towards the hearing aid (20) so as to implement disinfection by UVC irradiation.

10. An assembly comprising a cleaning device (1) according to any of claims 1 to 9, and a cleaning-liquid refilling unit (9) suitable for filling a reservoir (5) of the cleaning device (1), **characterized in that** the refilling unit (9) comprises a sleeve (923) suitable for cooperating with at least one cleaning cavity (3) of the cleaning device (1), so as to transfer the cleaning liquid from the refilling unit (9) to the reservoir (5) of the cleaning device (1).

11. The assembly according to claim 10, **characterized in that** the refilling unit (9) comprises, on the one hand, a vessel (90) provided with an opening (900) closed by a seal (901), and on the other hand, connection means which are suitable for perforating the seal (901) when they are connected to a cleaning cavity (3) of the cleaning device (1).

12. The assembly according to any of claims 10 and 11,
**characterized in that** the refilling unit (9) comprises a lid (92) having a channel (920), a perforator (91) suitable for perforating the seal (901) and means for connecting to the cleaning cavity (3), the lid (92) being movable relative to the vessel (90) between a preliminary position in which the perforator (91) is spaced apart from the opening (900), and a percussion position in which the perforator (91) perforates the seal (900) and releases the liquid through the channel (920).

13. The assembly according to any of claims 11 and 12,
**characterized in that** the perforator (91) defines a tube allowing the cleaning liquid contained in the vessel (90) to flow through the channel (920) of the lid (92) to the cleaning cavity (3).

14. The assembly according to any of claims 11 to 13, **characterized in that** the perforator (91) extends in the movement direction of the lid (92) and has two lateral faces (910) interconnected by a base (911) having a perforating tip (913) suitable for perforating the seal (901).
